# EUROPÄISCHE PATENTANMELDUNG

(11) **EP 4 603 248 A1**
(43) Veröffentlichungstag der Anmeldung: **20.08.2025**
(21) Anmeldenummer: 24157939.0
(22) Anmeldetag: 15.02.2024
(51) Int. Cl.: B28B 17/00, G01B 11/25, G01N 21/88, G01N 21/95, G01N 33/38, G06T 7/00, G06T 7/60, G06T 7/90, G01B 11/24, G06T 7/55

(54) **VERFAHREN ZUR ERFASSUNG EINER PRODUKTEINHEIT UND PRODUKTERFASSUNGSEINRICHTUNG**

(71) Anmelder: OMAG Maschinenbau GmbH, 26723 Emden (DE)
(72) Erfinder: WEETS, Jakob Focke, 26725 Emden (DE); IMMENGA, Immo Johann, 26721 Emden (DE); REBLIN, Jan Henning, 26721 Emden (DE)
(74) Vertreter: Jabbusch, Matthias

(57) **Zusammenfassung**

Verfahren zur Erfassung einer Produkteinheit (3, 3', 3", 3'", 3"", 3""`), bei dem diese entlang eines Produktförderweges (2) in einer Produktförderebene (A) an einem Produkterfassungsabschnitt (107) vorbeigeführt wird, dadurch gekennzeichnet, dass in dem Produkterfassungsabschnitt (107) zu jeder Produkteinheit (3, 3', 3", 3"`, 3"", 3""`) wenigstens drei unterschiedliche Abbildungen erzeugt werden, dass die Abbildungen wenigstens ein Höhenprofil der Betonsteine, eine Ausrichtung von Normalenvektoren der Betonsteine sowie eine Farbgebung der Betonsteine wiedergeben, dass die Abbildungen mit in einem Auswerteprogramm hinterlegten Daten abgeglichen und ausgewertet werden, und dass fehlerhafte Betonsteine einer Produkteinheit (3, 3', 3", 3'", 3"", 3""`) in einem Auswerteprogramm gespeichert oder markiert oder gespeichert und markiert werden.

## Beschreibung

Die Erfindung betrifft ein Verfahren zur Erfassung einer Produkteinheit aus Betonsteinen, bei dem diese entlang eines Produktförderweges in einer Produktförderebene an einem Produkterfassungsabschnitt vorbeigeführt werden. Weiter betrifft die Erfindung auch eine Produkterfassungseinrichtung für Betonsteine einer Produkteinheit aufweisend den Produktförderweg mit der Produktförderebene, dem in Förderrichtung der Produkteinheiten der Produkterfassungsabschnitt zugeordnet ist.

Um Produktfehler in vollautomatisierten bzw. weitestgehend automatisierten Produktionsprozessen zu vermeiden, sind zumeist umfassende Maßnahmen zur Qualitätssicherung erforderlich. Ein solcher Produktionsprozess ist die Herstellung von Betonsteinen, welche in unterschiedlichsten Formen und Varianten, unter anderem als Pflastersteine, Randsteine, Bordsteine, Platten, oder Riemchen, in sogenannten Betonsteinwerken produziert werden. Diese können nach dem Aushärten Löcher, Einschlüsse, Flecken, abgebrochene Kanten oder Risse in deren Materialstruktur aufweisen und müssen dementsprechend aussortiert werden. Da zumeist mehrere Betonsteine als eine Produkteinheit geformt und verarbeitet werden und in der Regel nur einzelne Teile bzw. Betonsteine einer solchen Produkteinheit Fehler aufweisen, erfolgt die Erfassung der Fehler und die Aussortierung betroffener Teile zumeist händisch von einem in dem Produktaustauschabschnitt tätigen Mitarbeiter. Aufgrund hoher körperlicher Belastung des Mitarbeiters kann es dabei vermehrt zu Fehlentscheidungen kommen, wobei fehlerhafte Produkteinheiten übersehen werden.

Aufgabe der Erfindung ist es daher Produkteinheiten oder Teile dieser mit entsprechenden Fehlern mit höherer Sicherheit zu erkennen und dadurch die Produktqualität zu erhöhen.

Die Lösung dieser Aufgabe erfolgt verfahrensmäßig mit den Merkmalen des Anspruchs 1. Vorrichtungsmäßig erfolgt die Lösung der Aufgabe gemäß den Merkmalen des Anspruchs 8. Weiterbildungen und Vorteilhafte Ausgestaltungen der Erfindung sind in den jeweils nachgeordneten Ansprüchen angegeben.

Das Verfahren zur Erfassung einer Produkteinheit aus Betonsteinen, bei dem diese entlang eines Produktförderweges in einer Produktförderebene an einem Produkterfassungsabschnitt vorbeigeführt wird, zeichnet sich erfindungsgemäß dadurch aus, dass in dem Produkterfassungsabschnitt zu jeder Produkteinheit wenigstens drei unterschiedliche Abbildungen erzeugt werden, dass die Abbildungen wenigstens ein Höhenprofil der Betonsteine, eine Ausrichtung von Normalenvektoren der Betonsteine sowie eine Farbgebung der Betonsteine wiedergeben, dass die Abbildungen mit in einem Auswerteprogramm hinterlegten Daten abgeglichen und ausgewertet werden und dass fehlerhafte Betonsteine einer Produkteinheit in dem Auswerteprogramm gespeichert und/oder markiert werden.

Mit den unterschiedlichen Abbildungen lassen sich verschiedene Abweichungen oder Fehler der Betonsteine einer Produkteinheit von einem Soll feststellen. Die einzelnen Abbildungen beziehen sich dabei jeweils auf einzelne Aspekte, die die Qualität der Betonsteine einer Produkteinheit betreffen. Die Kombination aus den unterschiedlichen Abbildungen ermöglicht dabei eine umfassende Überprüfung der Position, Form und Erscheinung der Produkteinheit bzw. der Betonsteine der Produkteinheit.

Für eine der Abbildungen der Produkteinheit wird nach einer ersten Weiterbildung zunächst ein Lichtbild der Produkteinheit erstellt, aus dem unter anderem die Farbgebung der Betonsteine ermittelt werden kann. Farbveränderungen können sich zum Beispiel durch abweichende Betonmischungen oder auch Kalkflecken ergeben und führen zu einem unerwünschten Erscheinungsbild.

Um das Lichtbild einer Produkteinheit mit in dem Auswerteprogramm hinterlegten Daten vergleichen zu können, wird dieses in weiterer Ausgestaltung unter definierten Lichtbedingungen in dem Produkterfassungsabschnitt angefertigt, wobei mittels Beleuchtung des Produkterfassungsabschnitts eine gleichbleibende Helligkeit der unmittelbaren Umgebung der Produkteinheit sichergestellt wird.

Weiter wird nach einer nächsten Weiterbildung wenigstens ein strukturiertes Lichtmuster auf die Produkteinheit projiziert und mit den aus dem Lichtmuster erhaltenen Werten wird dann wenigstens eine der Abbildungen berechnet. Erzeugt wird das wenigstens eine strukturierte Lichtmuster vorteilhafterweise mittels eines 3D-Laserscanners, wobei aus dem strukturierten Lichtmuster eine Geometrie der Betonsteine rekonstruiert wird. Die Geometrie der Betonsteine kann einem Benutzer dabei in Form einer Tiefenkarte oder in Form einer Punktwolke zur Verfügung gestellt werden.

Im Einzelnen wird aus der Tiefenkarte oder der Punktwolke dann von dem Auswerteprogramm zumindest das Höhenprofil der Betonsteine einer Produkteinheit und die Ausrichtung der Normalenvektoren der Betonsteine einer Produkteinheit berechnet. In den Abbildungen können das Höhenprofil und die Ausrichtung der Normalenvektoren in unterschiedlichen Farbabstufungen angezeigt werden. Gleiche Normalenvektoren und gleiche Höhen werden dann jeweils homogen in einem Farbton abgebildet. Insbesondere sind die Abbildungen des Höhenprofils und der Ausrichtung der Normalenvektoren mit unterschiedlichen Farbtönen bzw. Farbspektren hinterlegt, um eine Unterscheidung der Art der Abweichung bzw. des Fehlers der Betonsteine als Anwender einfacher unterscheiden zu können. Beispielsweise weist eine Darstellung des Höhenprofils einen roten Grundton in der Produktförderebene auf, der sich mit zunehmender Produkthöhe über der Produktförderebene entsprechend dem beispielsweise HSV-Farbband verändert. Demgegenüber weist die Darstellung der Normalenvektoren bevorzugt bläuliche Farbtöne bei lotrecht zu der Produktförderebene ausgerichteten Normalenvektoren auf. In Abhängigkeit davon, in welche Richtung und in welchem Winkel, die Normalenvektoren abweichend von deren lotrechter Ausrichtung geneigt sind, weisen diese dann eine andere Färbung auf. Insbesondere kann den in Förderrichtung der Produkteinheiten geneigten Normalenvektoren ein anderer Farbton als den quer zu der Förderrichtung geneigten Normalenvektoren zugeordnet sein.

Zusätzlich zu den Abbildungen des Höhenprofils und der Ausrichtung der Normalenvektoren kann aus dem wenigstens einen strukturierten Lichtmuster auch eine 2D-Darstellung erzeugt werden, die insbesondere eine Textur der Betonsteine hervorhebt und unterstützend zu dem Lichtbild bei einer Bewertung von Farbabweichungen, wie unter anderem Kalkflecken, herangezogen werden kann.

Die Auswertung kann dabei entweder vollautomatisiert durch Abgleich mit hinterlegten Daten des Auswerteprogramms erfolgen oder händisch, indem eine Person die aus den Daten mit dem Auswerteprogramm erzeugten Abbildungen betrachtet und fehlerhafte Betonsteine markiert. Hierbei kann von der Person zwischen den verschiedenen Ansichten gewechselt werden, um möglicherweise nur in bestimmten Abbildungen ersichtliche Fehler der Betonsteine sicher beurteilen zu können.

Insbesondere in Kombination mit einer durch eine Person durchgeführten Überprüfung kann das Auswerteprogramm auch lernend ausgelegt sein, so dass mit zunehmendem Datensatz des Auswerteprogramms eine verfeinerte, genauere Erkennung fehlerhafter Betonsteine ermöglicht ist.

Die optische Abtastung der Produkteinheit mit dem strukturierten Lichtmuster kann nach einer Weiterbildung mit der Beleuchtung des Produkterfassungsabschnitts gekoppelt sein, wobei der Produkterfassungsabschnitt, während das strukturierte Lichtmuster auf die Produkteinheit projiziert wird, verdunkelt wird.

Um die Position der einzelnen Betonsteine und der Produkteinheit exakt zu ermitteln, kann nach einer Weiterbildung vorgesehen sein, dass vor dem Erzeugen der Abbildungen eine Position einer Produktionsunterlage der Betonsteine erfasst wird. Die Position der einzelnen Betonsteine wird dann relativ zu der Produktionsunterlage ermittelt. Bei weiteren Stationen innerhalb des Produktförderweges, wie beispielsweise einem Aussortieren fehlerhafter Betonsteine, kann die Position der Betonsteine dann ebenso ausgehend von der Produktionsunterlage bestimmt werden.

Weiter betrifft die Erfindung auch eine Produkterfassungseinrichtung für Betonsteine einer Produkteinheit, insbesondere zur Durchführung des vorgenannten Verfahrens, aufweisend einen Produktförderweg mit einer Produktförderebene, dem in Förderrichtung der Produkteinheit ein Produkterfassungsabschnitt zugeordnet ist. Diese Vorrichtung zeichnet sich erfindungsgemäß dadurch aus, dass dem Produkterfassungsabschnitt ein Gestell zugeordnet ist, dass an dem Gestell wenigstens zwei voneinander verschiedene optische Erfassungssysteme angeordnet sind, dass die optischen Erfassungssysteme einen Erfassungsbereich aufweisen, mit dem diese von einer Produktauflageseite aus auf die Produktförderebene des Produktförderweges gerichtet sind, und dass die Erfassungsbereiche eine Breite in der Produktförderebene aufweisen, die den Produktförderweg vollständig abdeckt.

Die an dem Gestell gehaltenen optischen Erfassungssysteme können eine Produkteinheit mit Betonsteinen somit in einem definierten Abstand vollständig und kontaktlos erfassen. Einerseits ist damit sichergestellt, dass die Position einzelner Betonsteine zu der Produkteinheit, zu einer Produktionsunterlage der Produkteinheit oder dem Produktförderweg ermittelbar ist, und andererseits können mit den optischen Erfassungssystemen gewonnene Daten auf einfache Weise ausgewertet und mit hinterlegten Daten abgeglichen werden.

Um eine Verzerrung der zu den Produkteinheiten zu erstellenden Abbildungen zu minimieren, ist nach einer Weiterbildung vorgesehen, dass wenigstens eines der optischen Erfassungssysteme, insbesondere alle optischen Erfassungssysteme rechtwinklig zu dem Produktförderweg ausgerichtet sind, insbesondere mit ihren Erfassungsbereichen rechtwinklig zu dem Produktförderweg ausgerichtet sind. Weiter sind die optischen Erfassungssysteme vorteilhafterweise zudem mittig zu dem Förderweg ausgerichtet, das heißt beiderseits mit gleichem Abstand zu Rändern des Förderwegs in Förderrichtung dieses ausgerichtet.

Als eines der optischen Erfassungssysteme weist die Vorrichtung vorteilhafterweise wenigstens einen 3D-Laserscanner auf, mit dem wenigstens ein strukturiertes Lichtmuster erzeugbar ist. Das andere optische Erfassungssystem ist in weiterer Ausgestaltung eine 2D-Kamera zur Erstellung eines Lichtbildes. Von diesen ist dann nach einer Weiterbildung wenigstens die 2D-Kamera rechtwinklig zu dem Produktförderweg ausgerichtet. Der 3D-Laserscanner kann in weiterer Ausgestaltung in einem Winkel bis 20° zu der Produktförderebene geneigt sein, insbesondere in einem Winkel bis 15° zu der Produktförderebene geneigt sein, insbesondere in einem Winkel bis 10° zu der Produktförderebene geneigt sein., wobei daraus resultierende Verzerrungen eines erzeugten 3D-Bildes herausgerechnet werden.

Nach einer nächsten Weiterbildung ist das Gestell auf Vibrationspufferelementen gelagert oder die optischen Erfassungssysteme sind über Vibrationspufferelemente an dem Gestell gehalten. In einem Betonwerk oder einer Halle auftretende Erschütterungen übertragen sich somit nicht auf die Vorrichtung bzw. die optischen Erfassungssysteme der Vorrichtung und ermöglichen damit eine genauere Datenerfassung. Aus diesen Daten und daraus erstellten Abbildungen lassen sich dann auch kleinste Fehler und Abweichungen erkennen, so dass durch die Vibrationspufferelemente eine höhere Sensitivität der Vorrichtung erreicht ist.

Zum Schutz vor äußeren Umgebungseinflüssen, zum Beispiel Licht oder Staub, ist weiter vorgesehen, dass die optischen Erfassungssysteme innerhalb eines Gehäuses des Gestells angeordnet sind, wobei der Produktförderweg durch das Gehäuse geführt ist. Innerhalb des Gehäuses ist dann der Produkterfassungsabschnitt angeordnet, in dem durch das diesen umgebende Gehäuse möglichst gleichbleibende Bedingungen vorliegen.

Der Einfall von störendem Licht bei der optischen Erfassung der Produkteinheit in das Gehäuse kann in weiterer Ausgestaltung dadurch minimiert werden, dass das Gehäuse Gehäuseöffnungen für den Produktförderweg aufweist, die in ihrer Größe an eine zu erfassende Produkteinheit anpassbar sind. Solche verstellbaren Gehäuseöffnungen können dann entweder während der optischen Erfassung der Produkteinheit vollständig geschlossen werden oder weisen Elemente auf, die die Gehäuseöffnungen soweit verkleinern, dass diese ausgehend von der Produktauflageseite der Produktförderebene bis auf eine maximale Höhe der Produkteinheit abgesenkt werden. Produkteinheiten können dann auch bei teilweise geschlossener Gehäuseöffnung durch diese hindurchgefördert werden.

Um Staub und andere Partikel aus dem Produkterfassungsabschnitt, insbesondere aus dem Erfassungsbereich des Erfassungsabschnitts, fernzuhalten, weist das Gestell nach einer Weiterbildung wenigstens eine Belüftungsanlage auf, welche einen auf die optischen Erfassungssysteme bzw. deren Erfassungsbereiche gerichteten Luftstrom erzeugt. Störende Partikel werden dann in vorbestimmter Weise aus dem Produkterfassungsbereich bzw. dem Produkterfassungsabschnitt geblasen.

In weiterer Ausgestaltung ist in dem Gehäuse eine Beleuchtung angeordnet. Mit dieser lassen sich in dem Gehäuse stabile Lichtbedingungen erzeugen, welche vor allem die mit der 2D-Kamera erzeugten Abbildungen der Produkteinheit auswert- und vergleichbar machen.

Die Beleuchtung des Produkterfassungsabschnitts kann dabei mit einer mit dem 3D-Laserscanner gekoppelten Ausschalteinrichtung kombiniert sein. Für einen Zeitraum, in dem mit dem 3D-Laserscanner wenigstens ein strukturiertes Lichtmuster auf die Produkteinheit projiziert wird, kann die Beleuchtung dann ausgestaltet werden, um eine abgedunkelte Umgebung in dem Produkterfassungsabschnitt zu schaffen.

Ein Ausführungsbeispiel der Erfindung, aus dem sich weitere erfindungswesentliche Merkmale ergeben können, ist in der Zeichnung dargestellt. Gleiche Teile sind dabei in allen Figuren der Zeichnung mit gleichen Bezugszeichen versehen. Es Zeigen:
- Fig. 1:: eine perspektivische Darstellung der erfindungsgemäßen Vorrichtung;
- Fig. 2:: eine perspektivische Darstellung eines erfindungsgemäßen Speichers der Vorrichtung gemäß Fig. 1;
- Fig. 3:: eine perspektivische Darstellung eines Produktförderwegs mit zugeordneten Speichern der Vorrichtung gemäß Fig. 1 und Fig. 2;
- Fig. 4:: eine perspektivische Darstellung eines Ausschnitts eines erfindungsgemäßen Manipulators gemäß Fig. 1;
- Fig. 5:: eine perspektivische Darstellung eines Greifers des Manipulators mit einem erfindungsgemäßen Wechselaufsatz;
- Fig. 6:: eine perspektivische Darstellung eines erfindungsgemäßen Lagergestells für Wechselaufsätze gemäß Fig. 5;
- Fig. 7.:: eine perspektivische Darstellung des Lagergestells gemäß Fig. 6 in Bezug zu weiteren Komponenten der Vorrichtung;
- Fig. 8:: eine schematische Darstellung eines Ansaugbereichs eines erfindungsgemäßen Greifers;
- Fig. 9:: eine perspektivische Darstellung einer Produkterfassungseinrichtung der Vorrichtung gemäß Fig. 1; und
- Fig. 10a bis 10f:: eine schematische Darstellung eines Verfahrensablaufs bei einem Austausch eines fehlerhaften Teiles in Draufsicht auf einen Produktaustauschabschnitt der Vorrichtung gemäß Fig. 1 bis 4.

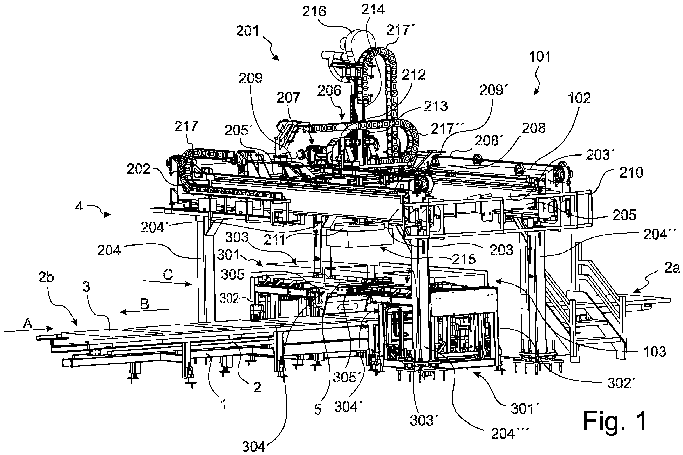

In Fig. 1 ist eine erfindungsgemäße Vorrichtung gezeigt, die eine Produktfördereinrichtung 1 aufweist. Die Produktfördereinrichtung 1 bildet einen Produktförderweg 2, der eine Produktzuführseite 2a und eine Produktabführseite 2b aufweist, wobei Produkteinheiten 3 durch die Produktfördereinrichtung 1 in einer Produktförderebene A von der Produktzuführseite 2a in Richtung der Produktabführseite 2b gefördert werden. In den Produktförderweg 2 sind eine Produkterfassungseinrichtung 101 und ein Manipulator 201 integriert, wobei der Manipulator 201 der Produkterfassungseinrichtung 101 in Förderrichtung B der Produkteinheiten 3 nachgeschaltet ist.

Von der Produkterfassungseinrichtung 101 ist in Fig. 1 lediglich ein diese umbauendes Gehäuse 102 ersichtlich, durch das die Produktfördereinrichtung 1 an entsprechenden Gehäuseöffnungen 103 hindurchgeführt ist.

Der Manipulator 201 bildet mit einem ersten Speicher 301 und einem zweiten Speicher 301' einen Produktaustauschabschnitt 4. Der Manipulator 201 weist ein portalartiges Maschinengestell 202 auf, dass die Produktfördereinrichtung 1 und die beiden Speicher 301, 301' überspannt. Dieses Maschinengestell 202 ist durch zwei in einer horizontalen Ebene parallel zueinander ausgerichtete Längsträger 203, 203', vier den Längsträgern 203, 203' endseitig zugeordnete, senkrechte Stützen 204, 204`, 204", 204‴ sowie zwei Querträgern 205, 205' gebildet, wobei die Querträger 205, 205` jeweils auf Höhe zweier der Stützen 204, 204', 204", 204‴ Enden der Längsträger 203, 203' miteinander verbinden.

An dem Maschinengestell 202 ist eine schienengebundene Laufkatze 206 an den zwei parallel zueinander ausgerichteten Längsträgern 203, 203' angeordnet. Entlang einer Längsachse der Längsträger 203, 203' ist die Laufkatze 206 bestimmungsgemäß oberhalb der Speicher 301, 301' und oberhalb einer Produktförderebene A der Produktfördereinrichtung 1 in einem rechten Winkel zu einer Förderrichtung B des Produktförderweges 2 verfahrbar. In Richtung der Längsträger 203, 203' wird die Laufkatze 206 mittels eines motorisch angetriebenen Riementriebs 207 geführt, der durch beidseitig zu der Laufkatze 206 an den Längsträgern 203, 203' angeordnete Riemen 208, 208' an Führungen 209, 209' der Laufkatze 206 angreift und diese entlang der Längsträger 203, 203' bewegt. Weiter ist den Enden der Längsträger 203, 203' ein Rammschutz 210 zugeordnet, der die Laufkatze 206 in ihrer Endlage vor Beschädigung von außen schützt, insbesondere der einem Antrieb des Riementriebs 207 gegenüberliegenden Endlage vor Beschädigung von außen schützt.

An einer den Produkteinheiten 3 zugewandten Unterseite der Laufkatze 206 ist ein Greifer 211 für Teile der Produkteinheiten 3 angeordnet, dem zwei an der Laufkatze 206 gehaltene Stellmotoren 212, 213 zugeordnet sind. Ein erster Stellmotor 212 bewegt den endseitig an einem Hubrahmen 214 gehaltenen Greifer 211 entlang einer vertikalen Achse. Der zweite Stellmotor 213 bewegt den Greifer 211 zusammen mit dem Hubrahmen 214 entlang einer horizontalen Achse, die parallel zu der Förderrichtung B des Produktförderweges 2 ausgerichtet ist.

Weiter weist die Laufkatze 206 eine Ansaugeinheit 215 auf, der ein entgegengesetzt zu dem Greifer 211 an dem Hubrahmen 214 angeordneter Unterdruckerzeuger 216 zugeordnet ist. Mit dem Unterdruckerzeuger 216 ist dann an der Ansaugeinheit 215 des Greifers 211 ein Unterdruck anlegbar, wobei der Hubrahmen 214 zwischen der Ansaugeinheit 215 und dem Unterdruckerzeuger 216 eine Ansaugleitung ausbildet. Um die Laufkatze 206 bzw. einzelne Komponenten dieser mit Strom und/oder Informationen, zum Beispiel zum Steuern des Greifers 211, zu versorgen, sind allen zueinander beweglichen Komponenten Energieketten 217, 217', 217" zugeordnet, die den Bewegungen der Laufkatze 206 oder Teilen dieser folgen.

Die Speicher 301, 301' sind zueinander baugleich und weisen jeweils ein Speichergestell 302, 302' mit einer Ablagefläche 303, 303' für jeweils eine Produkteinheit 3 auf. Die Speichergestelle 302, 302' sind seitlich zu dem Produktförderweg 2 einander gegenüberliegend zwischen den Stützen 204, 204', 204", 204‴ des Maschinengestells 202 angeordnet. Die Ablageflächen 303, 303' sind in einer gemeinsamen Austauschebene C angeordnet, die oberhalb der Produktförderebene A des Produktförderweges 2 angeordnet ist. Beide Speicher 301, 301' weisen Ein- und Ausschleusemittel 304, 304' mit jeweils einem Senktisch 305, 305' auf. Die Senktische 305, 305' sind von unterhalb der Produktförderebene A durch den Produktförderweg 2 hindurch bis in die Austauschebene C verfahrbar. Dabei erstrecken sich die Senktische 305, 305' ausgehend von den einander gegenüberliegenden Speichern 301, 301' gleichermaßen in Richtung einer Mittenachse des Produktförderweges 2 und weisen eine gemeinsame Hubsteuerung auf. In der Austauschebene C bilden die beiden Senktische 305, 305' zudem eine Auflagefläche für eine Produkteinheit 3 mit einem auszutauschenden Teil.

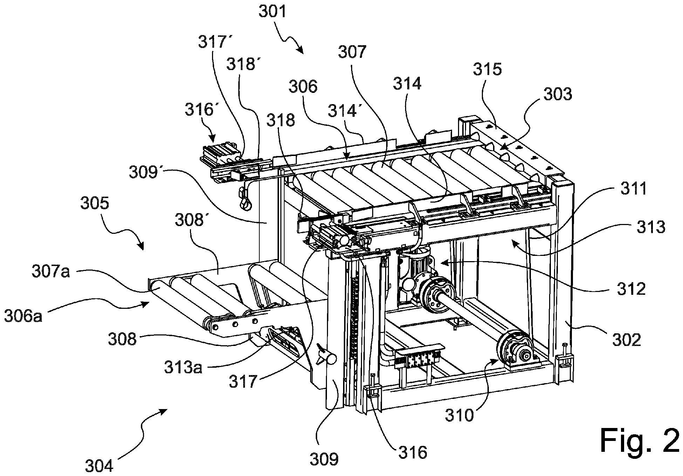

In Fig. 2 sind Details der Speicher 301, 301' anhand des Speichers 301 dargestellt. Dessen annähernd würfelförmiges Speichergestell 302 weist an seiner Oberseite die Ablagefläche 303 und an seiner der Produktfördereinrichtung 1 bestimmungsgemäß zuzuwendenden Frontseite den Senktisch 305 auf. Sowohl die Ablagefläche 303 als auch der Senktisch 305 sind durch eine Rollenbahn 306, 306a gebildet und weisen mit deren Drehachse parallel zu der Austauschebene C des Produktförderweges 2 ausgerichtete Rollen 307, 307a auf.

Die Rollen 307a des Senktisches 305 sind zwischen jeweils einem ersten Schenkel zweier Winkelbleche 308, 308' in einer horizontalen Senktischebene gehalten. Mit ihrem zweiten Schenkel sind die Winkelbleche 308, 308' in vertikal ausgerichteten Führungsschienen 309, 309' an der Frontseite des Speichergestells 302 geführt. Ein Riementrieb 310 zum Heben und Senken des Senktisches 305 ist ausgehend von einer Aufstellfläche der Vorrichtung unterhalb der Rollenbahn 306 der Ablagefläche 303 angeordnet und wirkt über einen Riemen 311 auf den Senktisch 305.

Das Ein- und Ausschleusemittel 304 umfasst neben dem Senktisch 305 und dessen Riementrieb 310 auch Kegelradgetriebemotoren 312, 313, 313a. Die Kegelradgetriebemotoren 313, 313a sind jeweils einer der Rollenbahnen 306, 306a zugeordnet und unterhalb der jeweiligen Rollenbahn 306, 306a angeordnet. Der Kegelradgetriebemotor 312 treibt den Riementrieb 310 an.

Wenn der Senktisch 305 mit der Senktischebene in die Austauschebene C mit der Ablagefläche 303 angehoben wird, bilden die Rollen 307, 307a der beiden Rollenbahnen 306, 306a eine gemeinsame Produktauflagefläche. Mit den auf die Rollenbahnen 306, 306a wirkenden Kegelradgetriebemotoren 313, 313a können die Rollen 307, 307a angetrieben werden und eine Produkteinheit 3 seitwärts zu dem Produktförderweg 2 von dem Senktisch 305 auf die Ablagefläche 303 oder von der Ablagefläche 303 auf den Senktisch 305 gefördert werden.

Um eine Produkteinheit 3 oder eine Produktunterlage 5 der Produkteinheit 3 auf der Ablagefläche 303 des Speichers 301 zu positionieren, sind den Rollen 307 der Rollenbahn 306 seitliche Führungselemente 314, 314` und gegenüberliegend zu der Frontseite ein Endanschlag 315 zugeordnet, die die Produkteinheit 3 auf der Ablagefläche 303 in bestimmter Weise zu dem Manipulator 201 ausrichten.

Eine auf einer durch die Senktische 305, 305' gebildeten Auflagefläche aufliegende Produkteinheit 3 wird mittels Positioniermitteln 316, 316` in einer bestimmten Position zu dem Manipulator 201 fixiert. Als Positioniermittel 316, 316' weist der Speicher 301 in Verlängerung der Führungsschienen 309, 309' in der Austauschebene C angeordnete, in einer horizontalen Ebene wirkende PneumatikZylinder 317, 317` mit an der Produkteinheit 3 anzulegenden, horizontal verschiebbaren Positionierhaltern 318, 318' auf. Der mit dem Senktisch 305 verfahrbare Kegelradgetriebemotor 313a ist mittels einer weiteren Energiekette 319 angebunden, um den Bewegungen des Senktisches 305 folgen zu können.

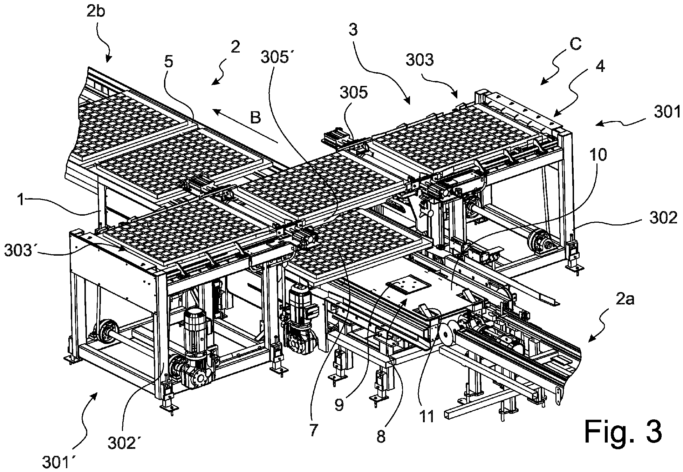

Der Fig. 3 ist neben dem Produktaustauschabschnitt 4 mit den beiden Speichern 301, 301' eine diesem in Förderrichtung B des Produktförderweges 2 vorgeschaltete Teile-Löse-Einrichtung 8 zu entnehmen. Diese Teile-Löse-Einrichtung 8 ist durch eine in die Produktfördereinrichtung 1 integrierte Anschlagplatte 9 gebildet. Die Anschlagplatte 9 ist ausgehend von einer Aufstellfläche der Vorrichtung mittig unterhalb der Produktförderebene A des Produktförderweges 2 angeordnet und kann in Richtung des Produktförderweges 2 bis oberhalb der Produktförderebene A angehoben werden oder gedrückt werden, insbesondere mittels Federkraft gegen eine Produkteinheit 3 gedrückt werden.

Beim Anheben dieser stößt die Anschlagplatte 9 dann gegen eine Produktunterlage 5 der Produkteinheit 3 und löst an der Produktunterlage 5 anhaftende Teile der Produkteinheit 3. Fehlerhafte Teile können dann mit dem Manipulator 201 von der Produktunterlage 5 angehoben und ausgetauscht werden, ohne dass der Manipulator 201 Kraft zum Lösen anhaftender Teile aufbringen muss.

Weiter zeigt Fig. 3 wie Produkteinheiten 3 mit fehlerfreien Teilen 7 in der Produktförderebene A unterhalb der Austauschebene C in Richtung der Produktabführseite 2b gefördert werden. Die Produktfördereinrichtung 1 weist zwischen der Produktzuführseite 2a und der Produktabführseite 2b zumindest im Bereich der Teile-Löse-Einrichtung 8 und des Produktaustauschabschnitt 4 Förderschlitten 10 auf, die unterhalb der Produktförderebene A in Förderrichtung B um wenigstens eine Länge einer Produkteinheit 3 oder einer Produktunterlage 5 der Produkteinheit 3 hin und zurück bewegt werden. An diesen Förderschlitten 10 sind einseitig wirkende Klinkenmitnehmer 11 ausgebildet, die die Produkteinheiten 3 oder Produktunterlagen 5 hintergreifen und in Förderrichtung B schieben und bei einer Zurückbewegung entgegengesetzt der Förderrichtung B von der Produkteinheit 3 oder der Produktunterlage 5 eingeklappt werden und unter diesen hindurchgleiten. Im Bereich der Senktische 305, 305' ist zwischen den Förderschlitten 10 in einer Endlage der Förderschlitten 10 eine Ausnehmung 12 ausgebildet, durch die die Senktische 305, 305` hindurchgehoben und -gesenkt werden können. Die Produkteinheiten 3 oder Produktunterlagen 5 liegen dabei nur mit ihren äußeren Ecken und auf einem Mittelsteg zwischen den Senktische 305, 305' auf, wobei die Senktische 305, 305' einen Abstand zueinander entsprechend dem Mittelsteg aufweisen.

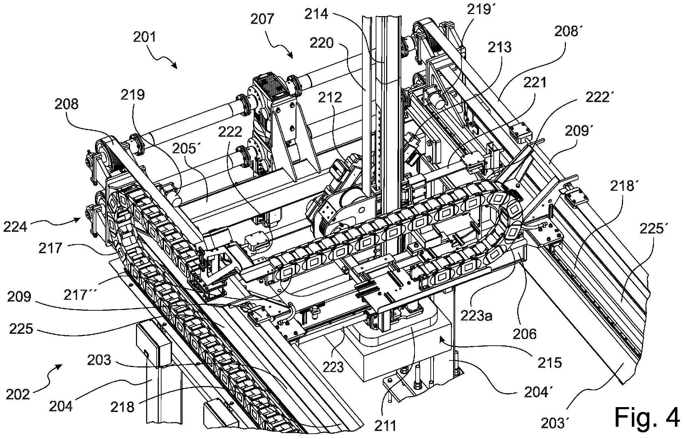

Fig. 4 zeigt einen Ausschnitt eines Manipulators 201' mit der Laufkatze 206. Aus diesem Ausschnitt ist zusätzlich zu Fig. 1 zu entnehmen, dass die Längsträger 203, 203` Kugelumlaufführungen 218, 218' aufweisen, auf denen die Laufkatze 206 beidseitig aufliegt. An Enden der Kugelumlaufführungen 218, 218' sind Endanschläge 219, 219' für die Laufkatze 206 angeordnet, die deren Bewegung begrenzen. Die Stellmotoren 212, 213 greifen an in der jeweiligen Bewegungsrichtung gespannte Riemen 220, 221 an, wobei zumindest auch dem Riemen 221 des Stellmotors 213 entsprechende Endanschläge 222, 222' zur Laufwegbegrenzung des Greifers 211 und der Kugelumlaufführungen 223, 223a zugeordnet sind.

Weiter zeigt Fig. 4 zusätzlich zu dem Riementrieb 207 mit den Riemen 208, 208' einen weiteren Riementrieb 224 mit den Riemen 225, 225', die zwischen den Riemen 208, 208' und der Austauschebene C an den Längsträgern 203, 203' angeordnet sind. Dieser Riementrieb 224 ist eine Vorbereitung des Manipulators 201' für eine Integration einer weiteren Laufkatze 206.

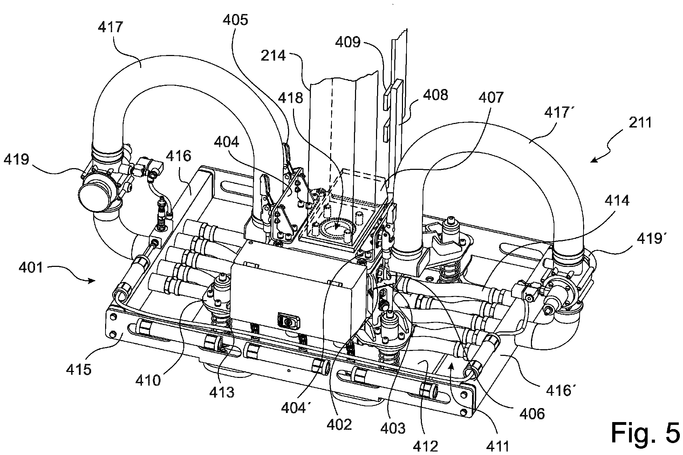

In Fig. 5 ist der Greifer 211 des Manipulators 201 gezeigt, der durch einen an dem Hubrahmen 214 gehaltenen Wechselaufsatz 401 gebildet ist. Der Hubrahmen 214 weist dazu endseitig einen Anbaurahmen 402 auf, der mit einem Montagestutzen 403 des Wechselaufsatzes 401 in Anlage gebracht ist. An dem Anbaurahmen 402 sind orthogonal zu einer Anlagefläche zwischen dem Montagestutzen 403 und dem Anbaurahmen 402 zwei parallel zueinander ausgerichtete Montagestege 404, 404' angeordnet, wobei jeder der Montagestege 404, 404' einem äußeren Rand des Anbaurahmens zugeordnet ist und zwei Hebelspanner 405 aufweist. Die Hebelspanner 405 hintergreifen jeweils einen Spannhaken 406 des Montagestutzens 403 und ziehen den Wechselaufsatz 401 damit an den Anbaurahmen 402 des Hubrahmens 214 heran.

Der Anbaurahmen 402 weist parallel zu der Anlagefläche des Montagestutzens 403 und des Anbaurahmens 402 zudem einen gegenüber dem Montagestutzen 403 vorkragenden Sicherungssteg 407 auf. An diesem Sicherungssteg 407 greift ein Sicherungshalter 408 an, der wiederum in ein Halteelement 409 der Laufkatze 206 eingehakt ist. Das Halteelement 409 ist parallel zu dem Hubrahmen 214 ausgerichtet und im Gegensatz zu dem Hubrahmen 214 positionsfest an der Laufkatze 206 angeordnet. Der an dem Hubrahmen 214 angreifende Sicherungshalter 408 und das Halteelement 409 blockieren den Hubrahmen 214 damit in vertikaler Bewegungsrichtung und ermöglichen bei z. B. Wartungsarbeiten einen sicheren Zugang für die Wartungsarbeiten durchführende Personen.

An dem Montagestutzen 403 ist ein gegenüber dem Montagestutzen 403 verbreiterter Tragrahmen 410 ausgebildet, der sich parallel zu der Anlagefläche des Montagestutzens 403 und des Anbaurahmens 402 sowie parallel zu einem Ansaugbereich 411 der Ansaugeinheit 215 erstreckt. Über den Tragrahmen 410 ist der Montagestutzen 403 mit einer Ansaugplatte 412 der Ansaugeinheit 215, die Ansaugeinheit 215 an dem Hubrahmen 214 haltend, verbunden. Zwischen der Ansaugplatte 412 und dem Tragrahmen 410 sind dazu an Ecken des Tragrahmens 410 und der Ansaugplatte 412 Federelemente 413 angeordnet, welche voneinander größtmöglich beabstandete Positionen an dem Tragrahmen 410 und der Ansaugplatte 412 aufweisen. Jedes dieser Federelemente 413 ist nur orthogonal zu der Ansaugplatte 412 stauchbar, so dass die Ansaugeinheit 215 in Ansaugrichtung einfedert, insbesondere nur parallel der Stauchrichtung der Federelemente 413 einfedert.

Dem Ansaugbereich 411 sind mehrere Ansaugkanäle 414 zugeordnet, welche in die Ansaugplatte 412 integriert sind, wobei der Ansaugbereich 411 in seinen Abmessungen durch die Ansaugplatte 412 begrenzt ist. Die Ansaugkanäle 414 sind rückwärtig zu dem Ansaugbereich 411 an der dem Tragrahmen 410 zugewandten Seite der Ansaugplatte 412 aus der Ansaugplatte 412 herausgeführt. An einem umlaufenden Begrenzungsrahmen 415 der Ansaugeinheit 215 sind die Ansaugkanäle 414 an einander gegenüberliegenden Leisten 416, 416' des Begrenzungsrahmens 415 zu jeweils einer Sammelleitung 417, 417` zusammengeführt. Diese Sammelleitungen 417, 417' münden in den Montagestutzen 403 und werden durch den Montagestutzen 403, über Ansaugöffnungen 418 in der Anlagefläche des Anbaurahmens 402 und des Montagestutzens 403 zu der innerhalb des Hubrahmens 214 ausgebildeten Ansaugleitung geleitet. Jeder Sammelleitung 417, 417` ist dabei ein Ventil 419, 419` zugeordnet, mit dem eine Luftströmung in der Sammelleitung 417, 417' steuer- und absperrbar ist.

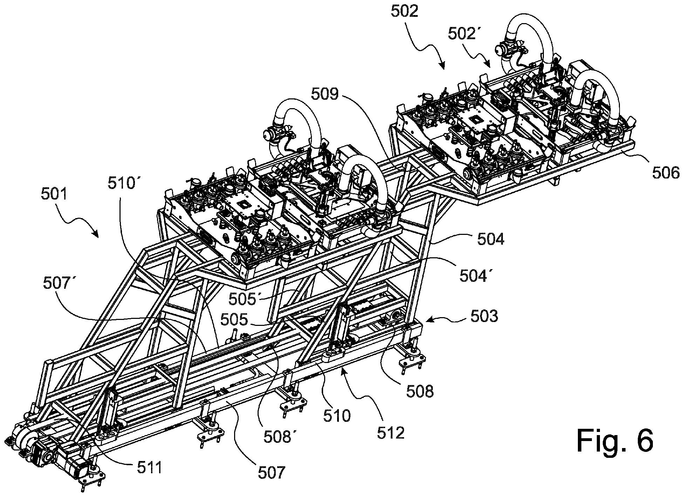

Die Fig. 6 zeigt ein Lagergestell 501 für Wechselaufsätze 401 mit zwei Lagerplätzen 502, 502'. Das Lagergestell 501 weist einen Gestellschlitten 503 auf, auf dem zwei parallel zueinander ausgerichtete senkrechte Stützen 504, 504' angeordnet sind. Den Stützen 504, 504` ist jeweils eine diagonale Strebe 505, 505' zugeordnet, die die jeweilige Stütze 504, 504` zu dem Gestellschlitten 503 abstützt und verstärkt. Die Stützen 504, 504` und die Streben 505, 505` sind an einer Lagerplatzaufnahmewanne 506 zusammengeführt und weisen an dem Gestellschlitten 503 deren größten Abstand zueinander auf.

Die Lagerplatzaufnahmewanne 506 weist die beiden Lagerplätze 502, 502' auf und bildet gegenüber den Stützen 504, 504' und den Streben 505, 505' an einer von den Streben 505, 505' abgewandten Seite der Stützen 504, 504' einen auskragenden Teil des Lagergestells 501. Die zwei Lagerplätze 502, 502` sind dabei vollständig dem auskragenden Teil zugeordnet und in einer gemeinsamen horizontalen Ebene angeordnet.

Der Gestellschlitten 503 ist an Schienen 507, 507` verfahrbar gelagert, wobei der Gestellschlitten 503 je Schiene 507, 507' einen Schlittenfuß 508, 508' aufweist. An den Schlittenfuß 508 sind die Stütze 504 und die Strebe 505 angebunden und an den Schlittenfuß 508` sind die Stütze 504` und die Strebe 505` angebunden. Sowohl die Schienen 507, 507` als auch die Schlittenfüße 508, 508', die Stützen 504, 504` und die Streben 505, 505' sind dabei parallel zu der jeweils anderen Schiene 507, 507', dem anderen Schlittenfuß 508, 508`, der anderen Stütze 504, 504' beziehungsweise der anderen Strebe 505, 505' angeordnet. Zwischen den Stützen 504, 504' und den Streben 505, 505' sind jeweils Querverbinder 509 angeordnet, die die zwei Stützen 504, 504' oder die zwei Streben 505, 505' fest miteinander verbinden.

Jede der Schienen 507, 507` weist einen horizontal ausgerichteten Steg 510, 510` auf, wobei die Stege 510, 510` mit deren freien Enden einander zugewandt sind. Die Schlittenfüße 508, 508` sind quer zu einer Längserstreckung der Schienen 507, 507` jeweils gegenüber den Stützen 504, 504' und den Streben 505, 505' verbreitert, so dass die Schlittenfüße 508, 508` in Richtung der Stege 510, 510' blockiert sind.

Zwischen den Schienen 507, 507' ist ein motorisch antreibbarer Riemen 511 gespannt, der sich parallel zu den Schienen 507, 507` über eine gesamte Wegstrecke des Lagergestells 501 erstreckt. Der Riemen 511 ist fest mit dem Lagergestell 501 verbunden und ermöglicht ein Verfahren des Lagergestells 501 entlang der durch die Schienen 507, 507' vorgegebenen Wegstrecke. Um das Lagergestell 501 in einer bestimmten Position zu der Wegstrecke, insbesondere in einer seiner Endlagen, zu sichern und zu fixieren, sind den Schienen 507, 507` Positioniermittel 512 zugeordnet.

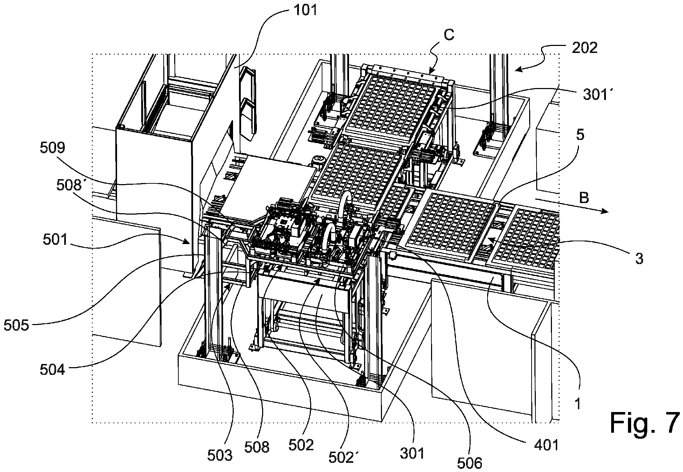

Die Schienen 507, 507` sind auf der Aufstellfläche der Vorrichtung montiert, wobei das Lagergestell 501 mit dem auskragenden Teil der Lagerplatzaufnahmewanne 506 in einen Arbeitsbereich des Manipulators 201 verfahrbar ist, wie dies in Fig. 7 dargestellt ist. In dem Arbeitsbereich des Manipulators 201 sind die Lagerplätze 502, 502` jetzt ausgehend von der Aufstellfläche der Vorrichtung oberhalb des Speichers 301 zwischen einer Austauschebene C für Teile 6, 7 der Produkteinheiten 3 und einer höheren Ebene, in der die Laufkatze 206 verfahrbar ist, angeordnet. Zum Austausch des Wechselaufsatzes 401 können jetzt beide Lagerplätze 502, 502` mit dem Manipulator 201 angesteuert werden, wobei einer der Lagerplätze 502, 502' einen Wechselaufsatz 401 von dem Manipulator 201 aufnimmt und von dem anderen Lagerplatz 502, 502' ein Wechselaufsatz 401 mit dem Manipulator 201 entnommen wird. Die Schienen 507, 507` sind dabei parallel zu der Förderrichtung B der Produktfördereinrichtung 1 angeordnet und weisen eine Länge auf, die es ermöglicht das Lagergestell 501 vollständig aus dem Arbeitsbereich des Manipulators 201 herauszuführen.

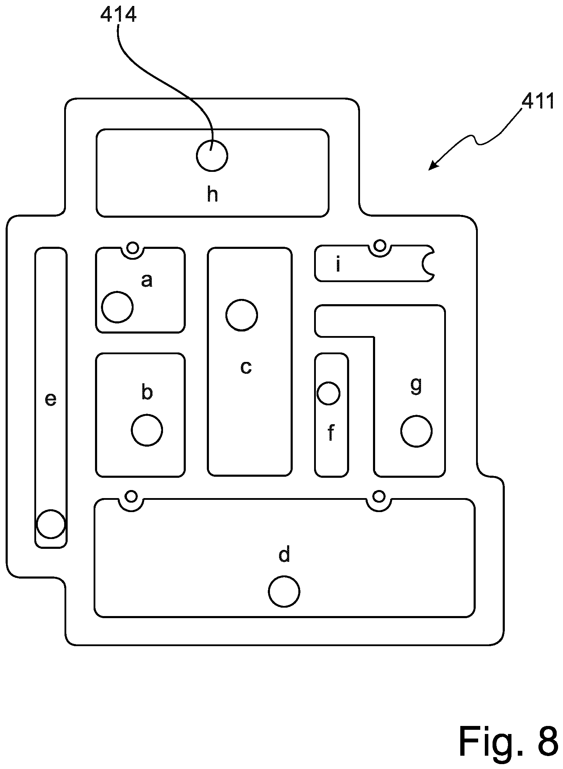

Fig. 8 zeigt schematisch einen Ansaugbereich 411 mit Ansaugabschnitten a, b, c, d, e, f, g, h, i einer Ansaugeinheit 215 des Greifers 211. Die Ansaugabschnitte a, b, c, d, e, f, g, h, i weisen jeweils einen Ansaugkanal 414 zum Anlegen eines Unterdrucks mit dem Unterdruckerzeuger 216 auf und haben jeweils unterschiedliche Formen, Größen oder Ausrichtungen. Zusammengelegt aktivierte Ansaugabschnitte a, b, c, d, e, f, g, h, i bilden Kombinationsansaugabschnitte, welche größere Flächen der Ansaugeinheit 215 abdecken. Beispiele für Kombinationsansaugabschnitte bilden die Ansaugabschnitte a, b, c, die Ansaugabschnitte a, b, c, h, die Ansaugabschnitte f, g, i, die Ansaugabschnitte a, b, c, f, g, i oder die Ansaugabschnitte a, b, c, d, f, g, i. Alle genannten Kombinationsansaugabschnitte bilden dabei größere Rechteckformen.

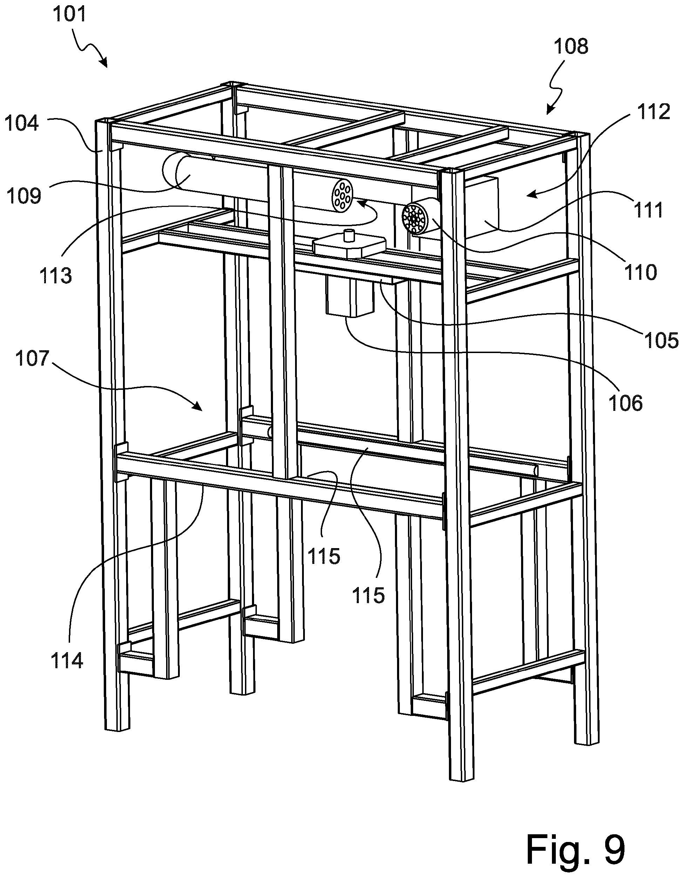

Aus Fig. 9 ist die Produkterfassungseinheit 101 ohne das Gehäuse 102 ersichtlich. Die Produkterfassungseinheit 101 weist ein Gestell 104 auf, an dem neben dem Gehäuse 102 weitere Komponenten der Produkterfassungseinheit 101 angeordnet sind. Diese Komponenten betreffen zwei optische Erfassungssysteme 105, 106, von denen das optische Erfassungssystem 105 ein 3D-Laserscanner und das optische Erfassungssystem 106 eine 2D-Kamera ist. Diese zwei optischen Erfassungssysteme 105, 106 sind gemäß dem aus Fig. 1 ersichtlichen, bestimmungsgemäßen Aufbau der Vorrichtung jeweils mittig oberhalb des Produktförderweges 2 angeordnet und rechtwinklig zu der Förderrichtung B der Produkteinheiten 3 auf eine gemäß Fig. 1 anzuordnende Produktfördereinrichtung 1 gerichtet. dabei weisen die optischen Erfassungssysteme 105, 106 einen Abstand zu der anzuordnenden Produktfördereinrichtung 1 auf, der entsprechend Erfassungsbereichen der optischen Erfassungssysteme 105, 106 so gewählt ist, dass der jeweilige Erfassungsbereich in der Produktförderebene A eine Breite wenigstens gleich der Produktfördereinrichtung 1 in der Förderrichtung B aufweist, um eine Produkteinheit 3 vollständig zu erfassen. Die optischen Erfassungssysteme 105, 106 und deren Erfassungsbereiche bilden dabei zusammen mit dem hindurchgeführten Produktförderweg 2 einen Produkterfassungsabschnitt 107 der Produkterfassungseinheit 101.

Den optischen Erfassungssystemen 105, 106 ist in Fig. 7 eine Belüftungsanlage 108 zugeordnet. Diese weist ein Belüftungsrohr 109 auf, dem in Strömungsrichtung von zur Belüftung angesaugter Luft ein Luftansaugstutzen 110, eine Filterbox 111 und ein Rohventilator 112 vorgeschaltet sind, wobei der Luftansaugstutzen 110 aus dem Gehäuse 102 herausgeführt ist. Das Belüftungsrohr 109 weist einen auf die optischen Erfassungssysteme 105, 106 und deren Erfassungsbereiche gerichteten Lüftungsauslass 113 auf, um den Produkterfassungsabschnitt 107 frei von Staub und anderen Verschmutzungen zu halten. An Querträgern 114 des Gestells 104 sind zudem Leuchtmittel 115 zur Beleuchtung des Produkterfassungsabschnitts 107 angeordnet.

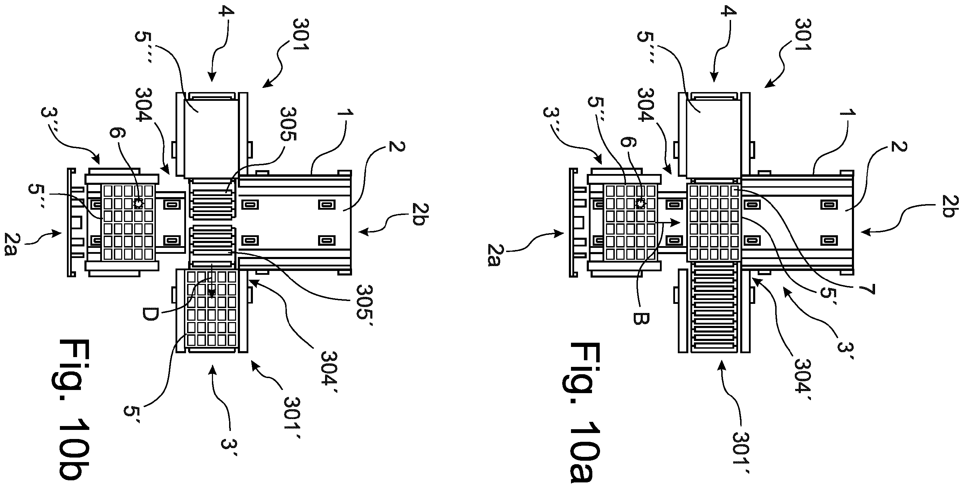

In den Fig. 10a bis Fig. 10f ist gezeigt, wie fehlerhafte Teile 6 einer Produkteinheit 3', 3", 3‴, 3ʺʺ, 3‴ʺ auf einer Produktunterlage 5', 5", 5‴, 5ʺʺ gemäß dem erfindungsgemäßen Verfahren ausgetauscht werden und wie die Produkteinheiten 3', 3", 3‴, 3ʺʺ, 3‴ʺ und Produktunterlagen 5', 5", 5‴, 5ʺʺ dabei gehandhabt, insbesondere verschoben, werden.

In Fig. 10a ist der Speicher 301 bereits mit einer Produktunterlage 5‴ belegt und auf der Produktfördereinrichtung 1 wird dem Produktaustauschabschnitt 4 auf einer Produktunterlage 5' eine Produkteinheit 3' mit fehlerfreien Teilen 7 zugeführt. Diese Produktunterlage 5' mit der Produkteinheit 3' wird, wie aus Fig. 10b ersichtlich ist, anschließend auf den Senktischen 305, 305' aufliegend in die Austauschebene C mit den Ablageflächen 303, 303' angehoben und mittels des Ein- und Ausschleusemittels 304` rechtwinklig zu der Förderrichtung B des Produktförderweges 2 in Richtung Pfeil D in den Speicher 301' eingefördert.

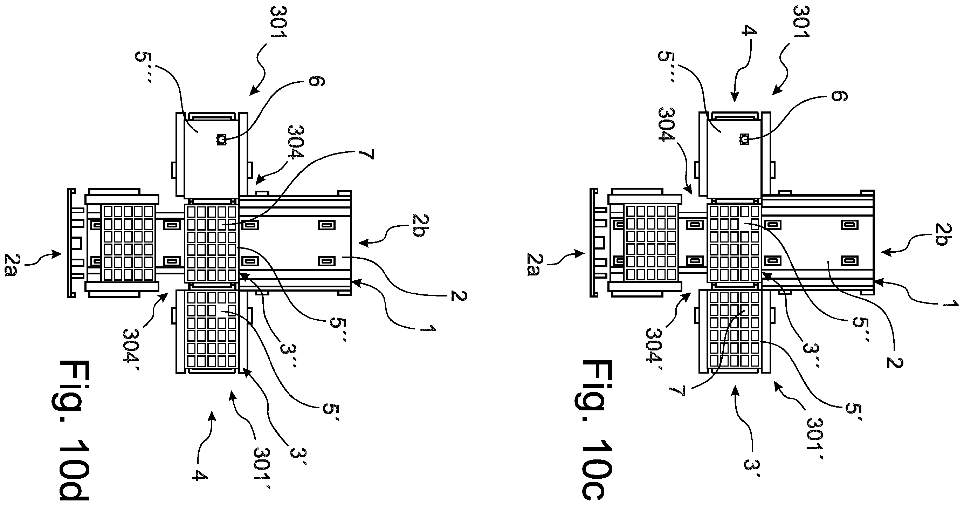

Auf die Produkteinheit 3' folgt eine Produktunterlage 5" einer Produkteinheit 3" mit einem fehlerhaften Teil 6, die in Fig. 10c in den Produktaustauschabschnitt 4 gefördert und mit den Senktischen 305, 305' in die Austauschebene C gehoben wurde. Weiter wurde mit dem Manipulator 201 das fehlerhafte Teil 6 aus der Produkteinheit 3" entnommen und positionsgetreu auf der Produktunterlage 5‴ abgelegt.

Nachdem das fehlerhafte Teil 6 auf der Produktunterlage 5‴ abgelegt wurde, wird der Manipulator 201 oberhalb der Produkteinheit 3' verfahren und ein fehlerfreies Teil 7 wird mit dem Manipulator 201 von der Produktunterlage 5' aufgenommen, angehoben und, wie in Fig. 10d mittels des Pfeils E angedeutet, zu der Produkteinheit 3" verfahren und an der Position des fehlerhaften Teiles 6 auf der Produktunterlage 5" abgelegt, so dass die Produkteinheit 3" vollständig aus fehlerfreien Teilen 7 gebildet ist.

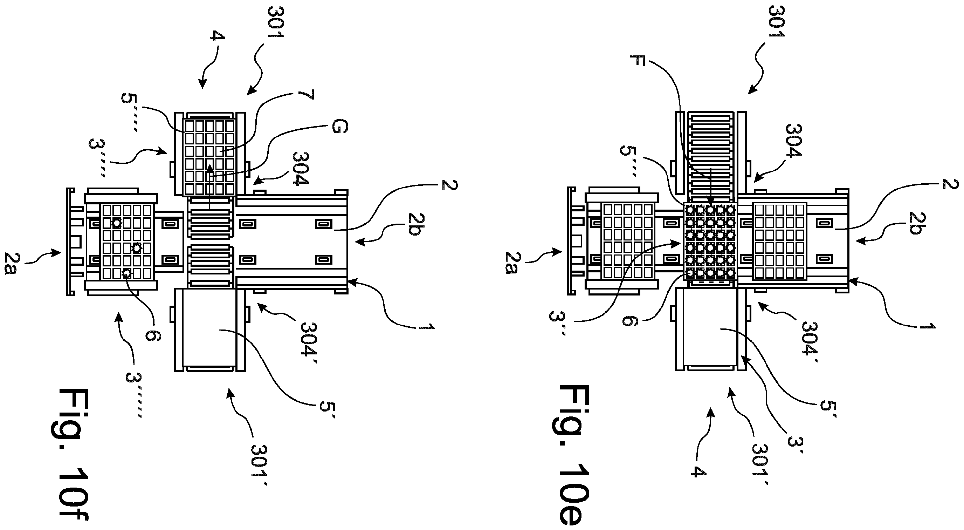

Sobald alle Positionen für fehlerhafte Teile 6 der Produktunterlage 5‴ belegt sind, wird diese gemäß Fig. 10e mit dem Ein- und Ausschleusemittel 304 in Richtung des Pfeiles F auf die Senktische 305, 305` gefördert und auf die Produktförderebene A der Produktfördereinrichtung 1 abgesenkt. Anschließend wird die Produktunterlage 5‴ einer aus fehlerhaften Teilen 6 gebildeten Produkteinheit 3‴ in Richtung des Pfeiles G zu der Produktabführseite 2b gefördert. Der Speicher 301' weist jetzt die leere Produktunterlage 5' auf.

In Fig. 10f wird jetzt der freie Speicher 301 entsprechend dem Pfeil G mit einer Produktunterlage 5ʺʺ einer Produkteinheit 3ʺʺ mit fehlerfreien Teilen 7 zugeführt, so dass die Speicher 301, 301' ihre Funktion tauschen. Fehlerhafte Teile 6 einer folgenden Produkteinheit 3‴ʺ werden jetzt auf der Produktunterlage 5' in dem Speicher 301' abgelegt und fehlerfreie Teile 7 von der Produkteinheit 3ʺʺ aus dem Speicher 301 entnommen.

## Patentansprüche

1. Verfahren zur Erfassung einer Produkteinheit (3, 3', 3", 3‴, 3ʺʺ, 3‴ʺ), bei dem diese entlang eines Produktförderweges (2) in einer Produktförderebene (A) an einem Produkterfassungsabschnitt (107) vorbeigeführt wird,
**dadurch gekennzeichnet,**
**dass** in dem Produkterfassungsabschnitt (107) zu jeder Produkteinheit (3, 3', 3", 3‴, 3ʺʺ, 3‴ʺ) wenigstens drei unterschiedliche Abbildungen erzeugt werden,
**dass** die Abbildungen wenigstens ein Höhenprofil der Betonsteine, eine Ausrichtung von Normalenvektoren der Betonsteine sowie eine Farbgebung der Betonsteine wiedergeben,
**dass** die Abbildungen mit in einem Auswerteprogramm hinterlegten Daten abgeglichen und ausgewertet werden, und
**dass** fehlerhafte Betonsteine einer Produkteinheit (3, 3', 3", 3‴, 3ʺʺ, 3‴ʺ) in einem Auswerteprogramm gespeichert oder markiert oder gespeichert und markiert werden.

2. Verfahren nach Anspruch 1, **dadurch gekennzeichnet, dass** die Farbgebung der Betonsteine aus einem Lichtbild ermittelt wird.

3. Verfahren nach Anspruch 2, **dadurch gekennzeichnet, dass** das Lichtbild bei definierten Lichtverhältnissen in dem Produkterfassungsabschnitt (107) angefertigt wird.

4. Verfahren nach einem der Ansprüche 1 bis 3, **dadurch gekennzeichnet, dass** wenigstens ein strukturiertes Lichtmuster auf die Produkteinheit (3, 3', 3", 3‴, 3"", 3‴ʺ) projiziert wird und mit den aus dem Lichtmuster erhaltenen Daten wenigstens eine der Abbildungen berechnet wird.

5. Verfahren nach Anspruch 4, **dadurch gekennzeichnet, dass** aus dem wenigstens einen strukturierten Lichtmuster zumindest das Höhenprofil der Betonsteine und die Ausrichtung der Normalenvektoren der Betonsteine berechnet wird.

6. Verfahren nach Anspruch 4 oder 5, **dadurch gekennzeichnet, dass** der Produkterfassungsabschnitt (107), während das strukturierte Lichtmuster auf die Produkteinheit (3, 3', 3", 3‴, 3ʺʺ, 3‴ʺ) projiziert wird, verdunkelt wird.

7. Verfahren nach einem der Ansprüche 1 bis 6, **dadurch gekennzeichnet, dass** vor dem Erzeugen der Abbildungen eine Position einer Produktionsunterlage der Betonsteine erfasst wird.

8. Produkterfassungseinrichtung (101) für Betonsteine einer Produkteinheit (3, 3', 3", 3‴, 3ʺʺ, 3‴ʺ), insbesondere zur Durchführung des Verfahrens gemäß einem der Ansprüche 1 bis 7, aufweisend einen Produktförderweg (2) mit einer Produktförderebene (A), dem in Förderrichtung (B) der Produkteinheiten (3, 3', 3", 3‴, 3ʺʺ, 3‴ʺ) ein Produkterfassungsabschnitt (107) zugeordnet ist,
**dadurch gekennzeichnet,**
**dass** dem Produkterfassungsabschnitt (107) ein Gestell (104) zugeordnet ist,
**dass** an dem Gestell (104) wenigstens zwei voneinander verschiedene optische Erfassungssysteme (105, 106) angeordnet sind,
**dass** die optischen Erfassungssysteme (105, 106) einen Erfassungsbereich aufweisen, mit dem diese von einer Produktauflageseite aus auf die Produktförderebene (A) des Produktförderweges (2) gerichtet sind, und
**dass** die Erfassungsbereiche eine Breite in der Produktförderebene (A) aufweisen, die die Förderebene des Produktförderwegs (2) vollständig abdeckt.

9. Produkterfassungseinrichtung (101) nach Anspruch 8, **dadurch gekennzeichnet, dass** wenigstens eines der optischen Erfassungssysteme (105, 106), insbesondere alle optischen Erfassungssysteme (105, 106), rechtwinklig zu dem Produktförderweg (2) ausgerichtet sind, insbesondere mit ihren Erfassungsbereichen rechtwinklig zu dem Produktförderweg (2) ausgerichtet sind.

10. Produkterfassungseinrichtung (101) nach einem der Ansprüche 8 oder 9, **dadurch gekennzeichnet, dass** eines der optischen Erfassungssysteme (105) ein 3D-Laserscanner ist.

11. Produkterfassungseinrichtung (101) nach einem der Ansprüche 8 bis 10, **dadurch gekennzeichnet, dass** eines der optischen Erfassungssysteme (106) eine 2D-Kamera ist.

12. Produkterfassungseinrichtung (101) nach einem der Ansprüche 8 bis 11, **dadurch gekennzeichnet, dass** das Gestell (104) auf Vibrationspufferelementen gelagert ist oder die optischen Erfassungssysteme (105, 106) über Vibrationspufferelemente an dem Gestell (104) gehalten sind.

13. Produkterfassungseinrichtung (101) nach einem der Ansprüche 8 bis 12, **dadurch gekennzeichnet, dass** die optischen Erfassungssysteme (105, 106) innerhalb eines Gehäuses (102) des Gestells (104) angeordnet sind, wobei der Produktförderweg (2) durch das Gehäuse (102) geführt ist.

14. Produkterfassungseinrichtung (101) nach Anspruch 13, **dadurch gekennzeichnet, dass** das Gehäuse (102) Gehäuseöffnungen (103) für den Produktförderweg (2) aufweist, die in ihrer Größe an eine zu erfassende Produkteinheit (3, 3', 3", 3‴, 3ʺʺ, 3‴ʺ) anpassbar sind.

15. Produkterfassungseinrichtung (101) nach einem der Ansprüche 8 bis 14, **dadurch gekennzeichnet, dass** das Gestell (104) wenigstens eine Belüftungsanlage (108) aufweist, welche ein auf die optischen Erfassungssysteme (105, 106) bzw. deren Erfassungsbereiche gerichteten Luftstrom erzeugt.

16. Produkterfassungseinrichtung (101) nach einem der Ansprüche 8 bis 15, **dadurch gekennzeichnet, dass** in dem Gehäuse (102) eine Beleuchtung angeordnet ist.
